# EUROPEAN PATENT SPECIFICATION

(11) **EP 1 865 106 B1**
(45) Date of publication and mention of the grant of the patent: **08.06.2011**
(21) Application number: 06713958.4
(22) Date of filing: 17.02.2006
(51) Int. Cl.: D21H 21/36, A61L 9/01, D21H 17/68

(54) **DEODORANT AND ANTIBACTERIAL FIBER PRODUCT**
DESODORIERENDES UND ANTIBAKTERIELLES FASERPRODUKT
PRODUIT FIBREUX DESODORISANT ET ANTIBACTERIEN

(30) Priority: 30.03.2005 JP 2005100001
(43) Date of publication of application: 12.12.2007
(73) Proprietor: KAO CORPORATION, Tokyo 103-8210 (JP)
(72) Inventor: ITOI, Takashi, c/o Kao Corp. Research Laboratories, Tochigi 321-3497 (JP); NOUKI, Ryuichi, c/o Kao Corp. Research Laboratories, Tochigi 321-3497 (JP)
(74) Representative: Vossius & Partner
(86) International application number: PCT/JP2006/302817
(87) International publication number: WO 2006/112128

(56) References cited:
- WO-A-2005/014059
- JP-A- 62 238 900
- JP-A- 2000 355 637
- JP-A- 2003 219 978
- JP-A- 2004 008 518
- JP-A- 2004 084 131
- JP-A- 2004 244 789
- JP-A- 2005 232 654
- US-A- 4 247 362

## Description

The present invention relates to a deodorant antimicrobial fibrous product and an absorbent article having the above deodorant antimicrobial fibrous product.

### Background Art

Various sheets utilizing adsorptivity of activated carbon have been known. For example, JP 9-173429A discloses a pulp sheet containing activated carbon fine particles that can be used in various deodorization applications. However, the sheet has no antimicrobial activity and is disadvantageous in that the activated carbon falls off easily.
WO2005/014059 discloses the use of metal substituted aluminosilicate particles for deodorisation. US-A-4 247 362 discloses the use of both soft- and hardwood fibers in paper sheets.

Various types of zeolite are known as a substance having antimicrobial activity as well as deodorizing activity. Zeolite can be supported on a sheet by kneading into a resin used to form a sheet. This method is inefficient because only part of the incorporated zeolite can be exposed on the surface of the sheet. When zeolite is adhered to the surface of a sheet, it is difficult to have a large amount of zeolite supported on a sheet made from a fibrous material such as paper formed by a wet papermaking technique because zeolite has poor fixability on a fibrous material like pulp. Moreover, even if zeolite may be incorporated in a large amount, efficient deodorant antimicrobial effects are not obtained. In other words, deodorant antimicrobial effects cannot be effectively exerted with a reduced amount of zeolite.

### Disclosure of the Invention

The present invention provides a deodorant antimicrobial fibrous product containing a cancrinite-like mineral containing an antimicrobial metal, a clay mineral, softwood bleached kraft pulp, and hardwood bleached kraft pulp as defined by claim 1.

The present invention also provides an absorbent article having the deodorant antimicrobial fibrous product of sheet form between a topsheet and an absorbent member, or within the absorbent member, or between the absorbent member and a backsheet as defined by claim 9.

### Brief Description of the Drawings

Fig. 1 is a perspective of an embodiment of the deodorant antimicrobial fibrous product according to the present invention.
Fig. 2 is a perspective of an absorbent member wrapped in the deodorant antimicrobial fibrous product of Fig. 1.
Fig. 3 is a transverse cross-section of an absorbent pad prepared in Examples and Comparative Examples.

### Detailed Description of the Invention

The present invention will be described based on its preferred embodiments. The deodorant antimicrobial fibrous product of the invention is a fibrous material having a deodorant antimicrobial agent adhered thereto. The deodorant antimicrobial agent includes a cancrinite-like mineral containing an antimicrobial metal (hereinafter referred to as a metal-substituted cancrinite-like mineral) and a clay mineral. The metal-substituted cancrinite-like mineral is a cancrinite-like mineral with its metal element displaced with an antimicrobial metal element. The fibrous material includes softwood (Nadelholz) bleached kraft pulp (hereinafter referred to as NBKP) and hardwood (Laubholz) bleached kraft pulp (hereinafter referred to as LBKP).

The cancrinite-like mineral has a structure similar to that of an aluminosilicate compound. The cancrinite-like mineral as referred to herein is one having at least one X-ray diffraction pattern selected from the group consisting of JCPDS (Joint Committee on Powder Diffraction Standards) Card Nos. 20-379, 20-743, 25-776, 25-1499, 25-1500, 30-1170, 31-1272, 34-176, 35-479, 35-653, 38-513, 38-514, 38-515, and 45-1373. One having an X-ray diffraction pattern with a main peak at d=0.365±0.015 nm is preferred.

As a result of the inventors' study, a metal-substituted cancrinite-like mineral has been found to have a broad deodorization spectrum and exhibit good deodorizing effects on a wide range of malodors including alkaline odors (e.g., ammonia, amines, and pyridines), acidic odors (e.g., lower fatty acids and mercaptans), and neutral odors (e.g., esters, ketones, and aldehydes). It has also been found to exhibit high antimicrobial activity. In short, a metal-substituted cancrinite-like mineral was proved to show high deodorant and antimicrobial activities even at a small amount of use. Besides, particles of a metal-substituted cancrinite-like mineral have been ascertained to have extremely good fixability or adhesion onto a fibrous material such as pulp owing to their shapes like a tetrapod or a star-burst (or a sea urchin).

Nevertheless, the present inventors have also revealed after studies that the metal-substituted cancrinite-like mineral reduces its essential deodorant antimicrobial activity when adhered to a fibrous material, particularly when incorporated into a slurry that is formed into sheeting by a wet papermaking process. Although the reason why is not necessarily clear, it is considered that various additives present in the system for fixing the metal-substituted cancrinite-like mineral to a fibrous material, such as a wet strength additive, may have adverse influences. After various studies contemplating prevention of reduction of deodorant antimicrobial activity of the metal-substituted cancrinite-like mineral, the inventors have found out that a combined use of a clay mineral with the metal-substituted cancrinite-like mineral can prevent the metal-substituted cancrinite-like mineral from reducing its deodorant antimicrobial activity when fixed to a fibrous material. The present invention has been completed based on these findings.

The findings make it possible to provide a deodorant antimicrobial fibrous product having a large quantity of a metal-substituted cancrinite-like mineral adhered thereto and thereby exhibiting extremely high deodorizing and antimicrobial performance. Sufficiently high deodorizing and antimicrobial performance is manifested even with a small amount of the metal-substituted cancrinite-like mineral to be adhered.

To prevent the metal-substituted cancrinite-like mineral from reducing the deodorizing and antimicrobial activity, the amount of the clay mineral in the deodorant antimicrobial fibrous product, while varying depending on the type of the clay mineral, is preferably 1% to 30% by weight, more preferably 1% to 10% by weight, based on the fibrous material. Examples of useful clay minerals include silicates other than cancrinite-like minerals, such as zeolite, sepiolite, and bentonite. Zeolite is particularly preferred for its high effect in preventing reduction of the deodorant antimicrobial effect of the metal-substituted cancrinite-like mineral. Various types of zeolite are known. Preferred of them is ZSM-5.

The amount of the clay mineral to be used relates to the amount of the metal-substituted cancrinite-like mineral to be adhered. Specifically, it is preferred to use the clay mineral in an amount of 100% to 800% by weight, more preferably 200% to 600% by weight, based on the metal-substituted cancrinite-like mineral to prevent reduction in deodorizing and antimicrobial activity of the metal-substituted cancrinite-like mineral.

On the other hand, the amount of the metal-substituted cancrinite-like mineral adhered to the fibrous material in the deodorant antimicrobial fibrous product is adjustable over a broad range as appropriate to the intended use of the fibrous product. For instance, the amount may be 0.01% to 20% by weight, preferably 0.2% to 10% by weight, based on the fibrous material.

The fibrous material that can be used in the deodorant antimicrobial fibrous product includes NBKP and LBKP. A combined use of these two types of fibrous materials imparts moderate strength and softness to the fibrous product. Using NBKP without LBKP tends to make the deodorant antimicrobial fibrous product harder, which can cause a manufacturing trouble when, for example, the fibrous product is further processed on a processing machine for manufacturing a product. Conversely, using LBKP without NBKP tends to result in the production of a deodorant antimicrobial fibrous product with reduced strength, which can also cause a manufacturing trouble on a processing machine such as paper breakage. From these considerations, the NBKP/LBKP weight ratio is preferably 95/5 to 50/50, more preferably 95/5 to 60/40.

In the case where the deodorant antimicrobial fibrous product of the present invention is manufactured by wet papermaking, it is desirable for successful sheet formation that an NBKP/LBKP blend is beaten to a controlled degree so as to have a CSF (Canadian Standard Freeness; JIS P8121) of 400 to 600 ml, preferably 450 to 600 ml.

The fibrous material that can be used in the deodorant antimicrobial fibrous product may be composed solely of NBKP and LBKP or may further contain rayon. The fibrous material may contain a small amount of heat fusible fiber of a thermoplastic resin.

A wet strength additive is preferably incorporated into the deodorant antimicrobial fibrous product of the invention, thereby to impart high wet strength to the fibrous product. When the fibrous product is used as a member constituting an absorbent article such as a disposable diaper, increasing wet strength of the fibrous product is advantageous in that the fibrous product hardly breaks even when wetted with urine, etc. To increase wet strength also means to increase dry strength. Therefore, the deodorant antimicrobial fibrous product with increased wet strength experiences less troubles that could occur where the fibrous product is fabricated into a final product on a processing line. For example, the deodorant antimicrobial fibrous product of the invention which has a sheet form preferably has a tensile strength of 400 cN/25 mm or higher in the MD in its dry state. The method of tensile strength measurement will be explained in Examples given later. The wet strength additive to be used is exemplified by a polyamideamine epichlorohydrin resin. The wet strength additive is preferably used in an amount of 0.01% to 5% by weight, more preferably 0.1% to 2.0% by weight, based on the fibrous material to obtain sufficient wet strength.

The metal-substituted cancrinite-like mineral is preferably one represented by compositional formula (1):

sM(1)ₓO_{y}·tM(2)₂O·Al₂O₃·uSiO₂·vRₘQₙ·wH₂O (1)

wherein M(1) represents an antimicrobial metal; M(2) represents at least one element selected from the group consisting of Na, K, and H; R represents at least one element selected from the group consisting of Na, K, Ca, and Mg; Q represents at least one atomic group selected from the group consisting of CO₃, SO₄, NO₃, OH, and Cl; and s, t, u, v, w, x, y, m, and n are numbers satisfying inequations: 0<s≤3; 0≤t≤3 (provided that s+t=0.5 to 3), 0.5≤u≤6, 0<v≤2, w≥0, 1≤x≤, 1≤y≤3, 1≤m≤2, and 1≤n≤3.

In formula (1), M(1) is Ag, Zn or Cu for their high deodorizing performance on sulfur-containing malodors. Ag is particularly preferred. M(1) may be one element or a combination of two or more elements. In the latter case, the term sM(1)ₓO_{y} is described for each term corresponding to each element. For example, when M(1) is a combination of metal elements D and D', sM(1)ₓO_{y} is represented by s₁Dₓ₁O_{y1}·s₂Dₓ₂O_{y2}, provided that x1+x2=x, y1+y2=y, and S₁+S₂=s. The same applies to the other terms.

M(2) is preferably Na and/or H in view of manifestation of high deodorizing ability and economy. From the same viewpoint, R is preferably at least one metal element selected from the group consisting of Na, Ca, and Mg, more preferably Na. Q is preferably CO₃ and/or NO₃ in terms of particle shape controllability.

s is preferably 0<s≤2, more preferably 0<s≤1, for the development of high deodorizing ability and from the economical viewpoint. t is preferably 0≤t≤2, more preferably 0≤t≤1, to maintain the pH of an aqueous dispersion of the metal-substituted cancrinite-like mineral (a 1 wt% aqueous dispersion, described *infra)* at an appropriate level. (s+t) is preferably 0.5 to 1.8, more preferably 0.6 to 1.5. u is preferably 0.5≤u≤5, more preferably 0.5≤u≤4, for the development of high deodorizing ability. v is preferably 0<v≤1.5, more preferably 0<v≤1, in view of particle shape controllability. w represents a water content (molar ratio) in the metal-substituted cancrinite-like mineral and varies according to the form of the metal-substituted cancrinite-like mineral, such as powdered or slurried. x and y are decided according to the combination of M(1) and O, and m and n are decided according to the combination of R and Q.

The metal-substituted cancrinite-like mineral preferably has a specific surface area of from 1 m²/g to less than 70 m²/g, more preferably 1 to 65 m²/g , even more preferably 30 to 65 m²/g. With a specific surface area falling within the preferred range, a cancrinite-like mineral can have an antimicrobial metal appropriately fixed or supported thereon to attain excellent deodorizing performance against sulfur-containing malodors. The specific surface area of the metal-substituted cancrinite-like mineral can be controlled by, for example, appropriately treating starting aluminosilicate particles as a raw material with an acid. The specific surface area is measured on a sample weighing 0.1 g using Flowsorb 2300 (from Shimadzu Corp.) and a nitrogen/helium (30/70 by volume) mixed adsorbate gas.

In order for the metal-substituted cancrinite-like mineral to have ensured deodorizing ability against sulfur-containing malodors, it is preferred that a 1 wt% aqueous dispersion of the metal-substituted cancrinite-like mineral has a pH of 7 or higher, more preferably 8 or higher, even more preferably 9 or higher. The pH of a 1 wt% aqueous dispersion of the metal-substituted cancrinite-like mineral is measured by the method described later.

The metal-substituted cancrinite-like mineral owes its deodorizing performance to the M(1) component's adsorbing sulfur-containing malodors. Therefore, it is preferred for obtaining excellent deodorizing power that much M(1) component is present in the vicinity of the surface of the metal-substituted cancrinite-like mineral. The surface concentration of the M(1) component is represented by the molar ratio of M(1) to Si atoms [M(1)/Si] or the molar ratio of M(1) to Al atoms [M(1)/Al], both as measured by ESCA. M(1)/Si is preferably 0.021 or greater, more preferably 0.040 or greater. M(1)/Al is preferably 0.025 or greater, more preferably 0.040 or greater. ESCA is carried out using a sample formed into a flake by a press and ESCA-1000 from Shimadzu Corp. The elements (M(1) element(s), Si, and Al) on the surface of the sample are analyzed, and the surface atom concentration ratio (molar ratio) is calculated from the peak areas of the elements.

The metal-substituted cancrinite-like mineral preferably has an average particle size of 0.1 to 1000 µm, more preferably 0.4 to 600 µm, even more preferably 1 to 100 µm, for increasing the speed of deodorization and improving powder fluidity. The average particle size is measured using, for example, a laser diffraction/scattering particle size distribution analyzer (LA-920, from Horiba, Ltd.), at a relative refraction index of 1.16.

The metal-substituted cancrinite-like mineral may be either amorphous or crystalline. It is preferably crystalline for higher performance of deodorizing sulfur-containing malodors. The metal-substituted cancrinite-like mineral is obtained as an aggregate of needle-like crystals, platy crystals, columnar crystals, etc. The crystals may flocculate to form spherical, tetrapod-shaped or lumpy aggregates, which may further agglomerate.

The term "needle-like crystals" as used herein denotes crystals having a thickness of 500 nm or smaller and an aspect ratio (length to thickness ratio) of 2.0 or greater. The term "platy crystals" as used herein means crystals having a thickness of 300 nm or smaller and an aspect ratio (major diameter to thickness ratio) of 2.0 or greater. The term "columnar crystals" as used herein refers to crystals having a thickness of 50 nm or greater and an aspect ratio (length to thickness ratio) of 1.0 or greater and smaller than 2.0.

The metal-substituted cancrinite-like mineral is preferably one prepared by a process including the step of acid treating a starting aluminosilicate the anhydride of which is represented by compositional formula: aM₂O·Al₂O₃·bSiO_{2·}CRₘQₙ (wherein M represents Na and/or K; R represents at least one element selected from the group consisting of Na, K, Ca, and Mg; Q represents at least one atomic group selected from the group consisting of CO₃, SO₄, NO₃, OH, and Cl; a, b, c, m, and n are numbers satisfying inequations: 0.5≤a≤3; 0.5≤b≤6, 0≤c≤2, 1≤m≤2, and 1≤n≤3) using 0 to 300 meq of an acid per 100 g of the starting aluminosilicate and the step of ion exchanging the starting aluminosilicate with an antimicrobial ion.

In formula above, M is preferably Na. When M is Na and K, aM₂O is represented by a'Na₂O·a"K₂O (provided that a'+a"=a). The same applies to the other terms. R is preferably at least one element selected from the group consisting of Na, Ca, and Mg, more preferably Na. Q is preferably CO₃ and/or NO₃. a is preferably 0.5≤a≤2.5, more preferably 0.5≤a≤2. b is preferably 0.5≤b≤5, more preferably 0.5≤b≤4. c is preferably 0<c≤1.5, more preferably 0<c≤1. m and n are decided according to the combination of R and Q.

It is preferred that the specific surface area of the starting aluminosilicate is nearly equal to that of the metal-substituted cancrinite-like mineral. It is also preferred that the average particle size of the starting aluminosilicate is nearly equal to that of the metal-substituted cancrinite-like mineral. The shape of the starting aluminosilicate is preferably the same as that of the metal-substituted cancrinite-like mineral, while not limited thereto.

The process of preparing the starting aluminosilicate is not particularly limited. To cite an example, the starting aluminosilicate can be obtained by the reaction between an alumina raw material and a silica raw material in an alkali solution in the presence of CO₃²⁻, SO₄²⁻, NO₃⁻, CI⁻, etc. Examples of the alumina raw material include aluminum oxide, aluminum hydroxide, and sodium aluminate. Sodium aluminate is particularly preferred. Examples of the silica raw material include silica sand, siliceous stone, water glass, sodium silicate, and silica sol. Water glass is particularly suitable. Also useful are raw materials serving as both an alumina raw material and a silica raw material, such as clay minerals, e.g., kaolin, montmorillonite, bentonite, mica, and talc; and aluminosilicate minerals, e.g., mullite. Examples of raw materials supplying CO₃²⁻ include carbonic acid gas, sodium carbonate, potassium carbonate, sodium potassium carbonate, calcium carbonate, and magnesium carbonate, with sodium carbonate being suitable. Examples of raw materials supplying SO₄²⁻ include sulfuric acid, sodium sulfate, potassium sulfate, and sodium potassium sulfate. Sulfuric acid or sodium sulfate is particularly preferred. Raw materials supplying NO₃⁻ include nitric acid, sodium nitrate, and potassium nitrate, with nitric acid or sodium nitrate being preferred. Raw materials supplying Cl⁻ include hydrochloric acid, sodium chloride, and potassium chloride, with hydrochloric acid or sodium chloride being preferred. Examples of the alkali of the alkali solution include oxides, e.g., sodium oxide and potassium oxide; hydroxides, e.g., sodium hydroxide and potassium hydroxide; carbonates, e.g., sodium carbonate, potassium carbonate, and sodium potassium carbonate; and hydrogencarbonates, e.g., sodium hydrogencarbonate and potassium hydrogencarbonate. Other alkalis that may be used if desired include oxides such as calcium oxide and magnesium oxide; hydroxides such as calcium hydroxide and magnesium hydroxide; carbonates such as calcium carbonate, magnesium carbonate, and dolomite; and hydrogencarbonates such as calcium hydrogencarbonate and magnesium hydrogencarbonate.

The starting aluminosilicate is obtainable by mixing the above-recited compounds in a predetermined ratio and causing the mixture to react. The mixing ratio is decided as appropriate to the desired composition of the starting aluminosilicate. The components composing the starting aluminosilicate being represented by M₂O, Al₂O₃, SiO₂, and RₘQₙ, preferred molar ratios of the components are such that M₂O/SiO₂ is 0.01 to 100, more preferably 0.05 to 80; Al₂O₃/SiO₂ is 0.01 to 10, more preferably 0.05 to 8; RₘQₙ/SiO₂ is 0.01 to 100, more preferably 0.05 to 80; and H₂O/M₂O is 0.01 to 100, more preferably 0.05 to 80.

The reaction temperature in the preparation of the starting aluminosilicate is preferably 15°C to 300°C, more preferably 60°C to 150°C, even more preferably 80°C to 130°C, to increase crystallinity of the starting aluminosilicate thereby to stabilize the form of the starting aluminosilicate, and to reduce chemical and pressure burdens on the reaction vessel. The reaction time is preferably 2 to 48 hours to ensure complete crystallization. The starting aluminosilicate is thus obtained usually in the form of an aqueous dispersion or slurry. The aqueous dispersion preferably has a solid concentration of 0.1% to 50% by weight.

The resulting starting aluminosilicate is subjected to acid treatment with 0 to 300 meq per 100 g of the starting aluminosilicate (hereinafter the amount of the acid is given in a unit meq/100 g). The acid treatment contemplates pH adjustment of the slurry when the M(1) component is fixed or supported on the starting aluminosilicate by ion exchange. The slurry is preferably adjusted to a pH of 7 or less, at which the M(1) component easily develops ion exchanging properties. The acid treatment also contemplates specific surface area adjustment. The amount of the acid to be used is preferably 6 to 300 meq/100 g, more preferably 5 to 250 meq/100 g, even more preferably 20 to 140 meq/100 g. To use 0 meq/100 g of an acid means that an acid treatment is not conducted. For instance, in the cases where the starting aluminosilicate has a specific surface area of from 1 m²/g to less than 70 m²/g, it does not need an acid treatment. The acid treatment is preferably effected using a strong acid such as hydrochloric acid, sulfuric acid or nitric acid. Hydrochloric acid or nitric acid is particularly preferred. The acid treatment is carried out by feeding an aqueous solution of the acid to the starting aluminosilicate either slowly or at a time to bring the acid and the aluminosilicate into contact with each other. The feed rate is preferably 0.01 to 100 ml/min, more preferably 0.1 to 10 ml/min, per 100 g of the starting aluminosilicate. The starting aluminosilicate to be acid treated is preferably in the form of a slurry. The solid matter concentration of the slurry is preferably 1% to 50% by weight to secure flowability of the reaction mixture and to prevent non-uniformity of the acid treatment thereby to improve the treating efficiency. The acid treating temperature is preferably 60°C to 150°C, more preferably 80°C to 120°C, to improve the specific surface area and to reduce the chemical and pressure burdens on the reaction vessel. The acid treatment may be conducted while stirring appropriately. The acid treatment is preferably performed for a period of 0.01 to 100 hours, more preferably 0.1 to 10 hours, from the contact between the acid and the starting aluminosilicate. After the acid treatment, the reaction mixture is preferably aged appropriately, for example, at 60°C to 150°C for about 0.1 to 1 hour.

The acid-treated aluminosilicate is subjected to ion exchange with an antimicrobial metal ion. Otherwise, a starting aluminosilicate with a desired specific surface area may be subjected directly to ion exchange without an acid treatment. The ion exchange is carried out by, for example, suspending the acid-treated aluminosilicate in water and adding a compound containing an antimicrobial metal (hereinafter "metal-containing compound") or an aqueous solution of a metal-containing compound, or immersing the starting aluminosilicate in an aqueous solution of a metal-containing compound. As mentioned above, the ion exchange does not need to be preceded by the acid treatment. The acid treatment and the ion exchange of the starting aluminosilicate may be carried out simultaneously by, for example, conducting the acid treatment in the co-presence of the metal-containing compound. The metal-containing compound is not particularly limited as long as it is water-soluble and contains a desired metal and is exemplified by a nitrate, sulfate or chloride containing a desired metal. The ion exchange is usually carried out while stirring an aqueous suspension of the starting aluminosilicate. To improve the ion exchange efficiency, the solid matter concentration of the aqueous suspension of the starting aluminosilicate is preferably 1% to 50% by weight. The temperature of the ion exchange system is not particularly limited but is preferably 20°C to 120°C, more preferably 80°C to 110°C. The ion exchange is conducted for a period of preferably 0.01 to 2 hours, more preferably 0.02 to 1 hour, from the contact between the starting aluminosilicate and the metal-containing compound. The starting aluminosilicate to metal-containing compound ratio in the ion exchange system is preferably 0.1 to 30 parts by weight, more preferably 0.2 to 10 parts by weight, even more preferably 0.5 to 5 parts by weight, of the metal-containing compound per 100 parts by weight of the starting aluminosilicate. The ion exchange is preferably followed by aging the reaction mixture appropriately, for example, at 60°C to 150°C for about 0.1 to 10 hours.

It is the best that the metal component in the metal-containing compound is fixed or supported on the cancrinite-like mineral through the above-described ion exchange. Nevertheless, it is possible to have the metal component of the metal-containing compound fixed or supported on the cancrinite-like mineral by immersion or precipitation in place of, or in addition to, ion exchange. In order to remove impurities, etc., the starting aluminosilicate may be washed in the production of the metal-substituted cancrinite-like mineral (a) after acquisition of the starting aluminosilicate, (b) after the acid treatment, and/or (c) after the ion exchange. It is particularly preferred that the washing is conducted in the final stage of the preparation of the starting aluminosilicate, e.g., after acquisition of the starting aluminosilicate and after ion exchange. The washing is carried out by, for example, filtering the aqueous suspension of the starting aluminosilicate and washing the filter cake with water. Examples of useful filters include, but are not limited to, a Buchner funnel and a filter press.

In the present invention, the above-described metal-substituted cancrinite-like mineral may be replaced with a cancrinite-like mineral represented by compositional formula (2) below in which part of M is substituted with an antimicrobial metal (hereinafter referred to as a second metal-substituted cancrinite-like mineral).

s₁M₂O·Al₂O₃·u₁SiO₂·v₁Rₘ₁Qₙ₁·w₁H₂O (2)

wherein M represents at least one element selected from the group consisting of Na, K, and H; R represents at least one element selected from the group consisting of Na, K, Ca, and Mg; Q represents at least one atomic group selected from the group consisting of CO₃, SO₄, NO₃, OH, and Cl; and s₁, u₁, v₁, w₁, m1, and n1 are numbers satisfying inequations: 0<s₁≤1, 1≤u₁≤50, 0<v₁≤2, w₁≥0, 1≤m1≤2, and 1≤n1≤2.

The second metal-substituted cancrinite-like mineral will then be described. The description on the aforementioned metal-substituted cancrinite-like mineral applies to the second one unless otherwise specified. The second metal-substituted cancrinite-like mineral is equivalent to the cancrinite-like mineral represented by compositional formula (2) with part of its M displaced with an antimicrobial metal. The amount of substitution with an antimicrobial metal in the cancrinite-like mineral represented by compositional formula (2) is preferably 0.1% to 30% by weight, more preferably 0.1% to 10% by weight, in view of development of desired deodorizing and antimicrobial activities and economical reasons. The amounts of these metals can be measured by X-ray fluorescence analysis.

The second metal-substituted cancrinite-like mineral preferably has part of its aluminum component leached out with an acid to create an amorphous state. Leaching of aluminum leaves a large number of micropores on the second metal-substituted cancrinite-like mineral to result in further increased deodorizing capability. Leaching of aluminum with an acid will be described later.

The second metal-substituted cancrinite-like mineral preferably has a specific surface area of 70 to 800 m²/g, more preferably 80 to 600 m²/g, even more preferably 100 to 500 m²/g, for the development of a moderate speed of deodorization and a broad deodorization spectrum.

The second metal-substituted cancrinite-like mineral preferably has an acid content of 20 meq/100 g or more, more preferably 100 meq/100 g or more, even more preferably 170 meq/100 g or more, for the improvement of alkaline malodor deodorizing ability. The term "acid content" refers to the total acid points in the second metal-substituted cancrinite-like mineral. The acid content is measured as follows. A sample weighing 0.5 g is stirred in 100 ml of a 0.01 mol/l NaOH aqueous solution for 1 hour. The resulting suspension is centrifuged (10000 rpm x 5 mins). A 25 ml portion of the superabsorbent liquid is titrated with 0.01 mol/l HNO₃ to obtain the amount of consumed NaOH, from which the acid content is calculated.

The second metal-substituted cancrinite-like mineral preferably has an average particle size of 1 to 500 µm, more preferably 1 to 300 µm, even more preferably 1 to 100 µm. With the average particle size falling within that range, a moderate speed of deodorization is obtained, and the second metal-substituted cancrinite-like mineral has good handling properties.

In the cancrinite-like mineral represented by compositional formula (2), M is preferably Na and/or H for the development of high deodorizing performance and from the economical consideration. When M is Na and H, s₁M₂O is represented by s'₁Na₂O·s'₂H₂O (wherein s'₁+s'₂=s₁). R is preferably Na for the same reason. Q is preferably CO₃ or NO₃ for ease of particle shape control.

s₁ is preferably 0<s₁≤0.5, more preferably 0<s₁≤0.25, for the improvement of alkaline malodor deodorizing ability. u₁ is preferably 1≤u₁≤40, more preferably 1≤u₁≤30, for the improvement of acidic malodor deodorizing ability v₁ is preferably 0<v₁≤1, more preferably 0<v₁≤0.6, even more preferably 0<v₁≤00.3, for the development of high deodorizing performance. w₁ is the water content (molar ratio) present in the precursor and varies depending on the form of the precursor, e.g., powdered or slurried. m1 and n1 are decided according to the combination of R and Q.

The second metal-substituted cancrinite-like mineral is suitably prepared by acid treating a starting aluminosilicate to form a cancrinite-like mineral, suspending the cancrinite-like mineral in water, and adding an aqueous solution of a water-soluble salt of an antimicrobial metal to the suspension to conduct ion exchange. In another suitable process, the second metal-substituted cancrinite-like mineral is prepared by carrying out the acid treatment of the starting aluminosilicate in the co-presence of the water-soluble salt of an antimicrobial metal thereby to conduct ion exchange simultaneously with the acid treatment.

The starting aluminosilicate that can be used to prepare the second metal-substituted cancrinite-like mineral is represented by compositional formula (3):

a₁M₂O·Al₂O₃·b₁SiO₂·c₁Rₘ₁Qₙ₁·zH₂O (3)

wherein M, R, Q, m1, and n1 are as defined for compositional formula (2); and a₁, b₁, c₁, and z are numbers satisfying inequations: 0.1≤a₁≤3, 0.2≤b₁≤6, 0<c₁≤2, and z≥0.
The process of preparing the starting aluminosilicate is not particularly limited. To cite an example, the starting aluminosilicate can be obtained by the reaction between an alumina raw material and a silica raw material in an alkali solution in the presence of CO₃²⁻, SO₄²⁻, NO₃⁻, Cl⁻, etc.

The acid treatment of the starting aluminosilicate for obtaining the cancrinite-like mineral is preferably effected using a strong acid such as hydrochloric acid, sulfuric acid or nitric acid. Hydrochloric acid or nitric acid is particularly preferred. By the acid treatment, not only M₂O and Rₘ₁Qₙ₁ present in the voids of the starting aluminosilicate but also part of the Al forming the skeleton are leached out. As a result, the specific surface area, pore volume, and acid points increase and, as previously stated, the finally obtained second metal-substituted cancrinite-like mineral becomes amorphous. The degree of the acid treatment is controlled as appropriate so that the finally obtained second metal-substituted cancrinite-like mineral may have desired properties. The acid treatment is carried out by feeding an aqueous solution of the acid to the starting aluminosilicate either slowly or at a time to bring the acid and the aluminosilicate into contact with each other. The feed rate is preferably 0.01 to 100 ml/min, more preferably 0.1 to 10 ml/min, per 100 g of the starting aluminosilicate.

After the acid treatment, the resulting cancrinite-like mineral is preferably aged at 60°C to 150°C for about 0.1 to 10 hours. The slurry is then filtered, and the filter cake is washed with water to remove an unnecessary ionic component. The resulting cancrinite-like mineral is suspended in water, the suspension heated to a predetermined temperature, and an aqueous solution of a water-soluble salt of an antimicrobial metal added thereto, followed by aging for a predetermined time, thereby to give the second metal-substituted cancrinite-like mineral.

The deodorant antimicrobial fibrous product of the present invention takes various forms depending on the method of making, including sheets, broken pieces of sheets, granules, and three-dimensional moldings. The deodorant antimicrobial fibrous products of such forms can be produced by a wet papermaking technique. The deodorant antimicrobial fibrous product of sheet form may be a single ply sheet containing the metal-substituted cancrinite-like mineral or a laminate sheet composed of a plurality of sheets. In the former case, the sheet is produced by wet papermaking using a slurry containing the fibrous material and the metal-substituted cancrinite-like mineral particles. The deodorant antimicrobial fibrous product of laminate sheet form is exemplified by the sheet illustrated in Fig. 1. The deodorant antimicrobial fibrous product of Fig. 1 is a laminate sheet composed of two rectangular pulp sheets of a size (a first pulp sheet 2 and a second pulp sheet 3) and a rectangular inner sheet 4 with a smaller width than the pulp sheets 2 and 3 interposed between the pulp sheets 2 and 3. The inner sheet 4 is a sheet of the fibrous material containing the metal-substituted cancrinite-like mineral particles, which is the same as the above-described single ply sheet. The inner sheet 4 is a paper sheet having the metal-substituted cancrinite-like mineral integrated therein by a papermaking technique and is held between the laterally middle portions of the two pulp sheets 2 and 3. The inner sheet 4 and the pulp sheets 2 and 3 are integrated by successively feeding the respective stocks to a paper machine.

The inner sheet 4 is absent in the lateral side portions 1a and 1b of the deodorant antimicrobial fibrous product 1. Namely, the lateral side portions 1a and 1b each have a double ply structure composed of the pulp sheets 2 and 3. With both the side portions of the deodorant fibrous product 1 sealed by joining the pulp sheets 2 and 3 along their lateral side portions, the metal-substituted cancrinite-like mineral is prevented from falling off from the side edges of the product 1. The width of the side portions 1a and 1b is preferably 0.1 to 20 cm, more preferably 1 to 6 cm, to secure prevention of the metal-substituted cancrinite-like mineral's falling off and to fulfill the function of the metal-substituted cancrinite-like mineral.

As previously stated, the sheet of the fibrous material containing the metal-substituted cancrinite-like mineral, i.e., the above-described single ply sheet or the inner sheet is produced by a wet papermaking technique using a slurry containing the fibrous material and particles of the metal-substituted cancrinite-like mineral. A flocculant is preferably added to the slurry to increase the amount of the metal-substituted cancrinite-like mineral fixed or adhered to the fibrous material. Examples of the flocculant include polyacrylamides and (meth)acrylic copolymers (molecular weight: 5,000,000 to 50,000,000). Polyacrylamides or (meth)acrylic copolymers (molecular weight: 10,000,000 to 30,000,000) are preferred. The amount of the flocculant to be added is preferably 0.01 % to 0.04% by weight, more preferably 0.01 to 0.035% by weight, based on the fibrous material, for sufficiently increasing the amount of the fixed metal-substituted cancrinite-like mineral and increasing the strength of the deodorant antimicrobial fibrous material of the invention.

The amount of the metal-substituted cancrinite-like mineral in the slurry is preferably 0.1 % to 10% by weight, more preferably 0.5% to 5.0% by weight, based on the fibrous material. The amount of the clay mineral is preferably 0.1% to 30% by weight, more preferably 0.1% to 10% by weight, based on the fibrous material. The flocculant is preferably added to increase the amount of the metal-substituted cancrinite-like mineral and the clay mineral to be fixed to the fibrous material. The amount of the flocculant is preferably 0.001% to 1.0% by weight, more preferably 0.001% to 0.04% by weight, based on the fibrous material from the standpoint of productivity. The amount of wet-strength additive is preferably 0.1% to 5% by weight, even preferably 0.1% to 2.0% by weight, based on the fibrous material. The concentration of the fibrous material in the slurry is preferably 0.5% to 5.0% by weight, more preferably 1.0% to 3.0% by weight.

The sheet obtained by wet papermaking, i.e., the above-described single ply sheet or the inner sheet has the metal-substituted cancrinite-like mineral fixed thereto at a fixing ratio of 25% or more. Good selection of a flocculant can result in achieving a fixing ratio as high as 50% or even higher. Such a high fixing ratio is largely owed to the shape of the metal-substituted cancrinite-like mineral as previously described. The grammage of the sheet is preferably 10 to 100 g/m², more preferably 13 to 70 g/m², which varies according to the intended use.

The deodorant antimicrobial fibrous product of sheet form may be cut or broken into small pieces. The deodorant antimicrobial fibrous product in the form of broken pieces is useful as, for example, a material for making up an absorbent member of an absorbent article described *infra.*

As previously described, the deodorant antimicrobial fibrous product of the present invention may take forms other than the sheet form, such as granules or three-dimensional moldings. A granular product can be obtainable by extruding a high concentration slurry of a fibrous material containing the metal-substituted cancrinite-like mineral from an extruder into strands, which are chopped into a predetermined size. Examples of the three-dimensional moldings include containers such as bottles, cups, and trays. Such three-dimensional moldings are conveniently produced by a pulp molding method. For the details of a pulp molding method, reference can be made to it, e.g., in commonly assigned WO99/42661.

The deodorant antimicrobial fibrous products according to the present invention are effective for antimicrobial deodorization in various applications. For example, applications of the deodorant antimicrobial fibrous product of sheet form or in the form of broken pieces of a sheet include materials for making absorbent articles such as disposable diapers, sanitary napkins and absorbent pads; wallpaper, bed sheets, closet liners, drawer liners, shoe cupboard liners, mats, insoles, masks, filters, and underlays for wrapping foods. The deodorant antimicrobial fibrous products of granular form such as beads or pellets are useful as, for example, pet deodorizers such as cat litter. The deodorant antimicrobial fibrous products of three-dimensional shape are useful as, for example, deodorant antimicrobial containers.

When the deodorant antimicrobial fibrous product of sheet form is used as a material for making an absorbent article, the sheet can be disposed, for example, between a topsheet and an absorbent member, or within an absorbent member, or between an absorbent member and a backsheet. Otherwise, the sheet can be used to wrap an absorbent material such as pulp and a superabsorbent polymer to make an absorbent member of an absorbent article. The topsheet is a sheet disposed on the skin facing side of an absorbent article and is generally liquid-permeable. The absorbent member is disposed between the topsheet and the backsheet and is generally liquid-retentive. The backsheet is disposed on the opposite side of the absorbent member to the topsheet, i.e., on the farther side of the absorbent member from the wearer's skin and is generally water-repellent or liquid-impermeable.

Fig. 2 illustrates an example of applying the deodorant antimicrobial sheet of the present invention to an absorbent article, in which an absorbent member of an absorbent article is wrapped in the deodorant antimicrobial sheet of the invention. The absorbent member 10 is composed of superabsorbent polymer particles and pulp fiber. The deodorant antimicrobial sheet 1 wraps around the absorbent member 10 and meets itself with its opposite side portions 1a and 1b overlapping each other. The absorbent member 10 as wrapped in this way is held between a topsheet (not shown) and a backsheet (not shown) to form an absorbent article. Thus, in this application, the deodorant antimicrobial sheet 1 is disposed between the topsheet and the absorbent member and also between the absorbent member and the backsheet of the absorbent article. In this application, an adsorbent for sulfur compounds such as hydrogen sulfide and mercaptans, which are typical malodorous substances, can be incorporated into any part of the absorbent article so that the metal of the metal-substituted cancrinite-like mineral may be effectively prevented from binding to such a sulfur compound. As a result, reduction of antimicrobial properties due to the binding between the metal and the sulfur compound is effectively prevented, which is advantageous for prolonged duration of antimicrobial properties. Improvement in deodorizing power of the metal-substituted cancrinite-like mineral also results. The sulfur compound adsorbent is typically exemplified by zinc oxide. The sulfur compound adsorbent can be adhered for example to the deodorant antimicrobial sheet of the invention or incorporated into the absorbent member.

### Example

The present invention will now be illustrated in greater detail with reference to Examples, but it should be understood that the invention is not construed as being limited thereto. Unless otherwise noted, all the percents are by weight.

### Example 1

In 1000 ml of ion exchanged water was dissolved 94 g of sodium hydroxide. Into the aqueous solution were mixed 130 g of nitric acid (61%) and 124 g of a sodium aluminate solution (Na₂O: 19.8%; Al₂O₃: 25.9%; H₂O: 54.3%). To the mixed solution was poured 127 g of water glass (Na₂O: 9.8%; SiO₂: 29.6%; H₂O: 60.6%) over 1 minute, followed by allowing the mixture to react at 100°C for 8 hours. After the reaction, the aluminosilicate particles thus formed were collected by filtration, washed, and dried at 105°C for 12 hours to give starting aluminosilicate particles in the form of powder. The resulting starting aluminosilicate particles were porous, spherical aggregates of needle-like crystals. X-ray diffraction with a powder X-ray diffractometer (RINT 2500, from Rigaku Corp.) showed a diffraction pattern corresponding to JCPDS No. 38-513. The resulting starting aluminosilicate particles had the shape of a star-burst, a composition of Na₂O·Al₂O₃·2SiO₂.0.4NaNO₃·0.7H₂O, and a specific surface area of 40 m²/g.

A hundred grams of the resulting starting aluminosilicate particles were suspended in 900 ml of ion exchanged water, and the suspension was maintained at 100°C. A predetermined amount of 61% nitric acid was added dropwise to the suspension while stirring at a rate of 1 ml/min to conduct acid treatment. An aqueous solution of 3.94 g of silver nitrate in 30 g of ion exchanged water was poured therein, followed by maintaining the mixture at 100°C for 1 hour to conduct ion exchange. The reaction mixture was filtered, and the filter cake was washed with water and dried at 105°C for 12 hours to yield a white Ag-substituted cancrinite-like mineral. The resulting Ag-substituted cancrinite-like mineral had the shape of a star-burst, a composition of 0.05Ag₂O·0.9Na₂O·Al₂O₃·2SiO₂·0.4NaNO₃·0.7H₂O, a specific surface area of 44.7 m²/g, and an average particle size of 8.3 µm. A 1% aqueous dispersion of the particles had a pH of 10.04. The surface Ag concentration was Ag/Si=0.075 and Ag/Al=0.070.

The resulting Ag-substituted cancrinite-like mineral was suspended in ion exchanged water. NBKP/LBKP blended pulp, synthetic zeolite 4A type (Silton, from Mizusawa Industrial Chemicals, Ltd.), a wet strength additive (Kaimen WS547, from Japan PMC Corp.), and a polyacrylamide flocculant (Accoflock A95, from Mitsui Aqua Polymer, Inc.) were mixed into the suspension to prepare a slurry. The blended pulp had been beaten to a CSF of 550 ml. The NBKP/LBKP weight ratio was 90/10.

The resulting slurry had a blended pulp concentration of 2%. The concentrations of the Ag-substituted cancrinite-like mineral, zeolite, wet strength agent, and polymeric flocculant based on the blended pulp content were 2%, 5%, 0.5%, and 0.025%, respectively.

The slurry was dewatered and shaped on a cylinder paper machine to obtain deodorant antimicrobial paper as a deodorant antimicrobial fibrous product. The grammage of the paper was 16 g/m². The deodorant antimicrobial paper contained 1% of the Ag-substituted cancrinite-like mineral, 2.5% of zeolite, 0.45% of the wet strength additive, and 0.018% of the flocculant each based on the blended pulp.

As illustrated in Fig. 3, a topsheet 11 formed of air-through nonwoven fabric weighing 20 g/m² was placed on one side of the resulting deodorant antimicrobial paper 12, and an absorbent member 13 made up of 350 g/m² of pulp and 120 g/m² of superabsorbent polymer particles was disposed on the other side. Both lateral side portions of the deodorant antimicrobial paper 12 and the absorbent member 13 were wrapped with absorbent paper 14 having a grammage of 15 g/m². A liquid impermeable backsheet 15 having a grammage of 15 g/m² was superposed on the back side of the wrapped absorbent member 13. There was thus obtained an absorbent pad of 150 mm in width and 400 mm in length. The nonwoven fabric, pulp, superabsorbent polymer, absorbent paper, and liquid impermeable sheet used here were the same as those used in an incontinence pad, Relief™ available from Kao Corp.

### Examples 2 and 3 and Comparative Examples 1 and 2

Deodorant antimicrobial paper and an absorbent pad were prepared in the same manner as in Example 1, except for changing the slurry composition as shown in Table 1 below.

### Evaluation

The fixing ratio of the Ag-substituted cancrinite-like mineral in the resulting deodorant antimicrobial paper was measured as follows. The deodorant antimicrobial paper was evaluated for softness, tensile strength (while dry and wet), productivity on a cylinder paper machine, and processability on a processing machine in accordance with the methods described below. Furthermore, the resulting absorbent pad was evaluated in terms of hydrogen sulfide deodorization rate and antibacterial activity in accordance with the following methods.

### (1) Fixing ratio of Ag-substituted cancrinite-like mineral

The fixing ratio of the Ag-substituted cancrinite-like mineral was calculated from the amount of the Ag-substituted cancrinite-like mineral in the slurry and that in the resulting deodorant antimicrobial paper. The latter amount was determined by wet disintegrating the deodorant antimicrobial paper, analyzing for Ag content on an ICP spectrometer, and calculating from the measured Ag content.

### (2) Softness

The softness was evaluated through (a) bulk softness measurement and (b) organoleptic evaluation. Each of those items was measured or evaluated by the methods described below.

### (a) Bulk softness

Five strip specimens were cut out of the deodorant antimicrobial paper with a width of 30 mm along the MD and a length of 150 mm along the CD. Both longitudinal ends of each specimen were joined with an about 9 mm overlap, and the overlap was stapled at both longitudinal ends thereof with a stapler (HD-10D with No. 10-1M staples, from Max) to make a cylindrical specimen of 45 mm in diameter and 30 mm in height. The cylindrical specimen was set on a compression tester (RTA-100, supplied by Orientec) and axially compressed at a rate of 10 mm/min. The maximum load applied during the compression was taken as a bulk softness in the MD. Bulk softness in the CD was obtained in the same manner except for using a sample measuring 30 mm in the CD and 150 mm in the MD cut out of the deodorant antimicrobial paper.

### (b) Organoleptic evaluation

A rectangular specimen measuring 300 mm along MD and 200 mm along CD was cut out of the deodorant antimicrobial paper. The specimen was put in a box so as to be hidden from view. A panel of 10 members touched the specimen in the box and rated its softness on the following scoring system, taking the softness of the specimen of Comparative Example 1 as a standard: 4=softer; 3=equal; 2=harder; 1=much harder. The softness of the specimen was graded "good" (average score=3.5 to 4), "medium" (average score=2.5 to 3.4) or "bad" (average score=2.4 or less).

### (2) Dry tensile strength

A strip specimen measuring 25 mm in width and 100 mm in length was cut out of the deodorant antimicrobial paper and quickly set on an universal compression tensile tester (RTM-25, from Orientec Co., Ltd.) to measure the strength at break at a pulling sped of 300 mm/min at an initial chuck distance of 50 mm. Measurement was taken in the machine direction (MD) and the direction perpendicular thereto (CD).

### (3) Wet tensile strength

A specimen of the deodorant antimicrobial paper of the same size as in the measurement of dry tensile strength was soaked in a large amount of water for 5 seconds and drained for 10 seconds. The strength at break of the wet specimen was measured in the same manner as for the dry tensile strength.

### (4) Productivity on cylinder paper machine

The deodorant antimicrobial paper was produced on a cylinder paper machine in a continuous manner. The state of the production process was monitored and graded "good" (no occurrence of troubles such as paper breakage or paper dusting), "medium" (occasional occurrence of troubles) or "bad" (frequent occurrence of troubles).

### (5) Processability on processing machine

Absorbent pads were continuously manufactured on an assembly line (processing machine). The state of production process was monitored and graded "good" (no production interruptions due to troubles such as paper breakage during conveying the deodorant antimicrobial paper), "medium" (occasional production interruptions) or "bad" (frequent production interruptions). Troubles such as paper breakage tend to occur during processing when the dry tensile strength (MD) of the deodorant antimicrobial paper is 400 cN/25 mm or less.

### (6) Hydrogen sulfide deodorization rate

In a 500 ml Erlenmeyer flask with ground glass stopper was put a cut piece of the absorbent pad measuring 100 mm by 100 mm. Into the flask was introduced hydrogen sulfide gas having a controlled concentration to result in an initial concentration of 3.5 ppm. Ten minutes later, the hydrogen sulfide gas concentration in the flask was measured with a gas detector tube (Hydrogen sulfide 4LT, from Gas Tech K.K.) to obtain a deodorization rate (measured value/initial concentration x 100).

### (7) Antibacterial activity

Artificial urine into which Escherichia coli was mixed was poured into the absorbent pad and incubated at 30°C for 24 hours. The absorbent pad was immersed in physiological saline in excess over its saturated absorption capacity and agitated, followed by filtration. The number of cells in the filtrate was measured. The antibacterial effect of the absorbent pad was graded based on the following criteria taking the results of Comparative Example 1 as a standard: minus (-)=growth of bacterium is suppressed compared with the standard; plus (+)=equal or accelerated growth compared with the standard.

**Table 1**

| | | | | Example | | | Comp. Example | |
|---|---|---|---|---|---|---|---|---|
| | | | | 1 | 2 | 3 | 1 | 2 |
| Slurry | Pulp | NBKP/LBKP | | 90/10 | 80/20 | 60/40 | 100/0 | 0/100 |
| | | CSF (ml) | | 550 | 550 | 600 | 450 | 700 |
| | | Concentration (%) | | 2 | 2 | 2 | 2 | 2 |
| | Composition (% to pulp) | Ag-substituted cancrinite-like mineral | | 2 | 1 | 2 | 2 | 2 |
| | | Zeolite | | 5 | 5 | 5 | 5 | 5 |
| | | Wet strength additive | | 0.5 | 0.5 | 0.5 | 0.5 | 0.5 |
| | | Flocculant | | 0.025 | 0.025 | 0.04 | 0.05 | 0.05 |
| Deodorant antimicrobial paper | Composition (% to pulp) | Ag-substituted cancrinite-like mineral | | 1 | 0.6 | 1.1 | 0.8 | 1 |
| | | Zeolite | | 2.5 | 3 | 2.75 | 2 | 2.5 |
| | | Wet strength additive | | 0.45 | 0.46 | 0.45 | 0.45 | 0.48 |
| | | Flocculant | | 0.018 | 0.021 | 0.031 | 0.037 | 0.041 |
| | Fixing ratio of Ag-substituted cancrinite-like mineral (%) | | | 50 | 60 | 55 | 40 | 50 |
| | Grammage (g/m²) | | | 16 | 16 | 16 | 16 | 16 |
| | Softness | Bulk Softness (g) | MD | 90 | 85 | 81 | 150 | 45 |
| | | | CD | 83 | 81 | 77 | 145 | 41 |
| | | Organoleptic softness | | good | good | good | medium | good |
| | Tensile strength (cN/25mm) | Dry. | MD | 900 | 830 | 650 | 1210 | 300 |
| | | | CD | 250 | 220 | 180 | 320 | 110 |
| | | Wet | MD | 350 | 250 | 280 | 400 | 160 |
| | | | CD | 80 | 70 | 75 | 90 | 40 |
| | Productivity on cylinder paper machine | | | good | good | good | medium | good |
| | Processability on processing machine | | | good | good | good | good | bad |
| Absorbent pad | Hydrogen sulfide deodorization rate (%) | | | 93 | 80 | 95 | 85 | 90 |
| | Antibacterial activity | | | - | - | - | - | - |

As is apparent from the results in Table 1, the deodorant antimicrobial paper of Examples (products of the present invention) are proved to be soft, have sufficient dry tensile strength, and be free from productivity problems on a paper machine and processability problems on a processing machine. In contrast, the deodorant antimicrobial paper of Comparative Example 1 is inferior in softness and can cause troubles during production on a paper machine.

### Industrial Applicability

The deodorant antimicrobial fibrous product according to the present invention has sufficiently high deodorizing and antimicrobial performance owing to the synergistic effect of a cancrinite-like mineral containing an antimicrobial metal and a clay mineral. Therefore, the absorbent article having the deodorant antimicrobial fibrous product of the invention effectively prevents generation of malodors from excreta and bacteria. When the cancrinite-like mineral and the clay mineral are combined with NBKP/LBKP, the deodorant antimicrobial fibrous product has a controlled paper strength and a soft hand.

## Claims

1. A deodorant antimicrobial fibrous product comprising:a cancrinite-like mineral containing an antimicrobial metal selected from Ag, Zn, Cu, or a combination thereof; a clay mineral; softwood bleached kraft pulp; and hardwood bleached kraft pulp; wherein the cancrinite-like mineral is one having at least one X-ray diffraction pattern selected from the group consisting of JCPDS (Joint Committee on Powder Diffraction Standards) Card Nos. 20-379, 20-743, 25-776, 25-1499, 25-1500, 30-1170, 31-1272, 34-176, 35-479, 35-653, 38-513, 38-514, 38-515, and 45-1373.

2. The deodorant antimicrobial fibrous product according to claim 1, wherein the weight ratio of the softwood bleached kraft pulp to hardwood bleached kraft pulp is 95/5 to 50/50.

3. The deodorant antimicrobial fibrous product according to claim 1 or 2, comprising 0.01% to 20% by weight of the cancrinite-like mineral and 1% to 30% by weight of the clay mineral based on a fibrous material comprising the softwood bleached kraft pulp and hardwood bleached kraft pulp.

4. The deodorant antimicrobial fibrous product according to any one of claims 1 to 3, comprising 0.01% to 0.04% by weight of a flocculant and 0.01% to 5% by weight of a wet strength additive based on a fibrous material comprising the softwood bleached kraft pulp and hardwood bleached kraft pulp.

5. The deodorant antimicrobial fibrous product according to any one of claims 1 to 4, wherein the cancrinite-like mineral is represented by compositional formula (1):
sM(1)ₓO_{y}·tM(2)₂O·Al₂O₃·uSiO₂·vRmQₙ·wH₂O (1)
wherein M(1) represents an antimicrobial metal; M(2) represents at least one element selected from the group consisting of Na, K, and H; R represents at least one element selected from the group consisting of Na, K, Ca, and Mg; Q represents at least one atomic group selected from the group consisting of CO₃, SO₄ NO₃, OH, and Cl; and s, t, u, v, w, x, y, m, and n are numbers satisfying inequations: 0<s≤3. 0≤5≤3 (provided that s+t=0.5 to 3), 0.5≤u≤6, 0<v≤2, w≥0, 1≤x≤2, 1≤y≤3, 1≤m≤2, and 1≤n≤3.

6. The deodorant antimicrobial fibrous product according to any one of claims 1 to 5, wherein the clay mineral is zeolite, sepiolite or bentonite.

7. The deodorant antimicrobial fibrous product according to any one of claims 1 to 6, which is in the form of as sheet or a piece thereof obtainable by cutting or breaking the sheet, granule, or three-dimensional molding, and is prepared by a wet papermaking technique. -

8. The deodorant antimicrobial fibrous product according to any one of claims 1 to 7, comprising a laminate comprising a sheet containing the cancrinite-like mineral and prepared by a wet papermaking technique and a pulp sheet superposed on each side of the sheet, the sheet being absent along at least one side portion of the laminate over the whole length of that side of the laminate.

9. An absorbent article comprising a top sheet, an absorbent member, a backsheet, and the deodorant antimicrobial fibrous product recited in claim 1, the deodorant antimicrobial fibrous product being in the form of a sheet and disposed between the topsheet and the absorbent member, within the absorbent member, or between the absorbent member and the backsheet.

## Patentansprüche

1. Ein deodorierendes antimikrobielles Faserprodukt, umfassend: ein Cancrinit-ähnliches Mineral, das ein aus Ag, Zn, Cu oder einer Kombination davon ausgewähltes antimikrobielles Metall; ein Tonmineral; gebleichten Kraftzellstoff aus Weichholz; und einen gebleichten Kraftzellstoff aus Hartholz enthält; wobei das Cancrinit-ähnliche Mineral eines ist, das mindestens ein Röntgenstrahlungsbeugungsmuster aufweist, das aus der Gruppe ausgewählt aus den JCPDS (Joint Committee on Powder Diffraction Standards) Kartennummern 20-379, 20-743, 25-776, 25-1499, 25-1500, 30-1170, 31-1272, 34-176, 35-479, 35-653, 38-513, 38-514, 38-515 und 45-1373 besteht.

2. Das deodorierende antimikrobielle Faserprodukt gemäß Anspruch 1, wobei das Gewichtsverhältnis des gebleichten Kraftzellstoffs aus Weichholz zum gebleichten Kraftzellstoff aus Hartholz 95/5 bis 50/50 beträgt.

3. Das deodorierende antimikrobielle Faserprodukt gemäß Anspruch 1 oder 2, umfassend 0,01 Gew.-% bis 20 Gew.-% des Cancrinit-ähnlichen Minerals und 1 Gew.-% bis 30 Gew.-% des Tonminerals, bezogen auf ein Fasermaterial, welches den gebleichten Kraftzellstoff aus Weichholz und den gebleichten Kraftzellstoff aus Hartholz umfasst.

4. Das deodorierende antimikrobielle Faserprodukt gemäß einem der Ansprüche 1 bis 3, umfassend 0,01 Gew.-% bis 0,04 Gew.-% eines Flockungsmittels und 0,01 Gew.-% bis 5 Gew.-% eines Nassfestzusatzmittels, bezogen auf ein Fasermaterial, welches den gebleichten Kraftzellstoff aus Weichholz und den gebleichten Kraftzellstoff aus Hartholz umfasst.

5. Das deodorierende antimikrobielle Faserprodukt gemäß einem der Ansprüche 1 bis 4, wobei das Cancrinit-ähnliche Mineral durch die Formel (1) mit der Zusammensetzung
sM(1)ₓO_{y}•tM(2)₂O•Al₂O₃•uSiO₂•vRₘQₙ•wH₂O (1)
dargestellt ist,
wobei M(1) ein antimikrobielles Metall darstellt; M(2) mindestens ein Element, ausgewählt aus der aus Na, K und H bestehenden Gruppe darstellt; R mindestens ein Element, ausgewählt aus der aus Na, K, Ca und Mg bestehenden Gruppe darstellt; Q mindestens eine atomare Gruppe, ausgewählt aus der aus CO₃, SO₄, NO₃, OH und Cl bestehenden Gruppe darstellt; und s, t, u, v, w, x, y, m und n Zahlen sind, die den Ungleichungen 0<s≤3, 0≤t≤3 (mit der Maßgabe, dass s+t=0,5 bis 3 ist), 0,5≤u≤6, 0<v≤2, w≥0, 1≤x≤2, 1≤y≤3, 1≤m≤2 und 1≤n≤3 genügen.

6. Das deodorierende antimikrobielle Faserprodukt gemäß einem der Ansprüche 1 bis 5, wobei das Tonmineral Zeolith, Sepiolit oder Bentonit ist.

7. Das deodorierende antimikrobielle Faserprodukt gemäß einem der Ansprüche 1 bis 6, welches in Form einer Bahn oder eines durch Schneiden oder Zerbrechen der Bahn erhältlichen Stückes davon, eines Granulats oder eines dreidimensionalen Formlings vorliegt und durch eine Technik der nassen Papierherstellung hergestellt wird.

8. Das deodorierende antimikrobielle Faserprodukt gemäß einem der Ansprüche 1 bis 7, umfassend ein Laminat, das eine Bahn, die das Cancrinit-ähnliche Mineral enthält und durch eine Technik der nassfesten Papierherstellung hergestellt wird, und einen Zellstoffbogen umfasst, welcher auf jeder Seite der Bahn aufliegt, wobei die Bahn entlang mindestens eines Seitenbereichs des Laminats über die Gesamtlänge dieser Laminatseite abwesend ist.

9. Ein absorbierender Gegenstand, umfassend eine obere Bahn, ein absorbierendes Element, eine rückwärtige Bahn und das deodorierende antimikrobielle Faserprodukt gemäß Anspruch 1, wobei das deodorierende antimikrobielle Faserprodukt in Form einer Bahn vorliegt und zwischen der oberen Bahn und dem absorbierenden Element, innerhalb des absorbierenden Elements, oder zwischen dem absorbierenden Element und der rückwärtigen Bahn liegt.

## Revendications

1. Produit fibreux antimicrobien désodorisant comprenant : une matière minérale semblable à la cancrinite contenant un métal antimicrobien choisi parmi Ag, Zn, Cu ou une combinaison de ceux-ci ; une matière minérale d'argile ; une pâte à papier kraft blanchie de résineux ; et une pâte à papier kraft blanchie de feuillus ; dans lequel la matière minérale semblable à la cancrinite est une présentant au moins un motif de diffraction aux rayons X choisi dans le groupe consistant en les numéros de cartes JCPDS (Joint Committee on Powder Diffraction Standards) N° 20-379, 20-743, 25-776, 25-1499, 25-1500, 30-1170, 31-1272, 34-176, 35-479, 35-653, 38-513, 38-514, 38-515 et 45-1373.

2. Produit fibreux antimicrobien désodorisant selon la revendication 1, dans lequel le rapport massique de la pâte à papier kraft blanchie de résineux à la pâte à papier kraft blanchie de feuillus est de 95/5 à 50/50.

3. Produit fibreux antimicrobien désodorisant selon la revendication 1 ou 2 comprenant de 0,01 % à 20 % en masse de la matière minérale semblable à la cancrinite et de 1 % à 30 % en masse de la matière minérale d'argile sur la base d'un matériau fibreux comprenant la pâte à papier kraft blanchie de résineux et la pâte à papier kraft blanchie de feuillus.

4. Produit fibreux antimicrobien désodorisant selon l'une quelconque des revendications 1 à 3 comprenant de 0,01 % à 0,04 % en masse d'un floculant et de 0,01 % à 5 % en masse d'un additif de résistance à l'état humide sur la base d'un matériau fibreux comprenant la pâte à papier kraft blanchie de résineux et la pâte à papier kraft blanchie de feuillus.

5. Produit fibreux antimicrobien désodorisant selon l'une quelconque des revendications 1 à 4, dans lequel la matière minérale semblable à la cancrinite est représentée par la formule de composition (I) :
sM(1)ₓO_{y}•tM(2)₂O•Al₂O₃•uSiO₂•vRₘQₙ•wH₂O (I)
dans laquelle M(1) représente un métal antimicrobien ; M(2) représente au moins un élément choisi dans le groupe constitué de Na, K et H ; R représente au moins un élément choisi dans le groupe constitué de Na, K, Ca et Mg ; Q représente au moins un groupe atomique choisi dans le groupe constitué de CO₃, SO₄, NO₃, OH et Cl ; et s, t, u, v, w, x, y, m et n sont des nombres satisfaisant les inéquations : 0<s≤3, 0≤t≤3 (à condition que s+t =0,5 à 3), 0,5≤u≤6, 0<v≤2, w≥0, 1≤×≤2, 1≤y≤3, 1≤m≤2 et 1≤n≤3.

6. Produit fibreux antimicrobien désodorisant selon l'une quelconque des revendications 1 à 5, dans lequel la matière minérale d'argile est une zéolithe, une sépiolite ou une bentonite.

7. Produit fibreux antimicrobien désodorisant selon l'une quelconque des revendications 1 à 6, lequel est dans la forme d'une feuille ou d'une pièce de celle-ci pouvant être obtenue en découpant ou en cassant la feuille, d'un granulé ou d'un moulage tridimensionnel, et est préparé par une technique de fabrication de papier par voie humide.

8. Produit fibreux antimicrobien désodorisant selon l'une quelconque des revendications 1 à 7 comprenant un stratifié comprenant une feuille contenant la matière minérale semblable à la cancritine et préparée par une technique de fabrication de papier par voie humide et une feuille de pâte à papier superposée sur chaque côté de la feuille, la feuille étant absente le long d'au moins une partie de côté du stratifié sur la longueur totale de ce côté du stratifié.

9. Article absorbant comprenant une feuille supérieure, un élément absorbant, une feuille arrière et le produit fibreux antimicrobien désodorisant cité dans la revendication 1, le produit fibreux antimicrobien désodorisant étant dans la forme d'une feuille et étant disposé entre la feuille supérieure et l'élément absorbant, à l'intérieur de l'élément absorbant, ou entre l'élément absorbant et la feuille arrière.
